(19) Europäisches Patentamt

European Patent Office

Office européen des brevets

(11) EP 0 565 197 B1

(12) EUROPEAN PATENT SPECIFICATION

(45) Date of publication and mention
of the grant of the patent:
**27.08.1997 Bulletin 1997/35**

(51) Int Cl.$^6$: **C07C 2/58**

(21) Application number: **93201015.0**

(22) Date of filing: **06.04.1993**

(54) **Process for upgrading a paraffinic feedstock**

Verfahren zur Verbesserung einer paraffinischen Ausgangsmischung

Procédé pour la valorisation d'une charge paraffinique

(84) Designated Contracting States:
**AT BE CH DE DK ES FR GB GR IE IT LI NL PT SE**

(30) Priority: **08.04.1992 EP 92201016**

(43) Date of publication of application:
**13.10.1993 Bulletin 1993/41**

(73) Proprietor: **SHELL INTERNATIONALE
RESEARCH MAATSCHAPPIJ B.V.
2596 HR Den Haag (NL)**

(72) Inventors:
• **Peferoen, Danny Gaston René
  NL-1031 CM Amsterdam (NL)**
• **Gilson, Jean-Pierre
  NL-1031 CM Amsterdam (NL)**
• **Sie, Swan Tiong
  NL-1031 CM Amsterdam (NL)**
• **De Jong, Krijn Pieter
  NL-1031 CM Amsterdam (NL)**
• **Stork, Willem Hartman Jurriaan
  NL-1031 CM Amsterdam (NL)**

(56) References cited:
**EP-A- 0 174 836          WO-A-92/04977
FR-A- 2 631 956          GB-A- 733 753**

**Description**

The present invention relates to a process for upgrading a paraffinic feedstock. More specifically the invention relates to a process for alkylation of a paraffinic feedstock by the condensation of paraffins with olefins.

The production of highly branched hydrocarbons such as trimethylpentanes is important by virtue of their use as gasoline blending components of high octane number. Traditional production of highly branched hydrocarbons is by condensation of isobutane with light olefins, usually butenes but sometimes mixtures of propene, butenes and possibly pentenes using large quantities of conventional strong liquid acid catalysts, such as hydrofluoric or sulphuric acids. An emulsion of immiscible acid and hydrocarbon is agitated to emulsify the catalyst and reactant and refrigerated to control the highly exothermic reaction. By fine control of a complex interrelation of process variables high quality alkylate production may be maintained. The acid is recycled after use. It is desirable to use a process which is less hazardous and environmentally more acceptable.

Processes have been proposed to overcome these problems by using solid acids as catalysts. However paraffin olefin condensation yields both desired alkylate and undesired oligomerisation product. When catalysing alkylation with solid acids it has been found that the selectivity for alkylate over oligomerisation product is less than that obtained with liquid acids. Moreover oligomerisation products are thought to cause the observed progressive deactivation of the catalyst. Regeneration techniques are known for removing hydrocarbonaceous deposits from solid catalysts and restoring catalyst activity. Nevertheless using known regeneration techniques for example raising the catalyst to elevated temperatures and oxidising the deposits, the alkylation must be interrupted and reactor conditions altered causing lost production time.

In US Patent No. 3,706,814 a process is disclosed for alkylation of isoparaffins using acidic zeolite catalysts and supplying to the reactor paraffin and olefin in a ratio of between 15 and 30, the concentration of unreacted olefin in the reactor maintained at less than 12 mole percent. Specifically the process is operated in a continuous stirred reactor using a zeolite Y catalyst. However, from the results of the experiments severe deactivation is seen to occur already at low catalyst ages.

In the process of French patent No. 2,631,956 isobutane and butene are reacted over zeolite-beta catalyst in an upflow fixed bed reactor. Product analysis at 1 hour and 4 hours time on stream shows a decrease in the percentage-olefin conversion level in the reactor with time on stream.

There is a need for an alkylation process which selectively yields highly branched alkylate at an acceptable rate for prolonged periods on stream. It has now suprisingly been discovered that by operation of a solid acid catalysed alkylation process at a high extent of internal circulation of liquid reactor content the rate of catalyst deactivation may be significantly decreased.

Furthermore it has suprisingly been discovered that by such operation particularly beneficial selectivity for highly branched paraffins is achieved for the duration of the catalyst activity.

Accordingly the present invention provides a process for upgrading a paraffinic feedstock comprising:

a) supplying the feedstock and an olefins-containing stream at a paraffin to olefin ratio greater than 2 v/v to a reactor containing a solid acid catalyst and

b) removing the upgraded product wherein the process is operated in an internal circulation reactor at an olefin space velocity in the range from 0.01 to 10 kg/kg.h, a temperature of less than 150°C and a variance in residence time distribution of liquid reactor contents of greater than 0.25 such that the process has an olefin conversion of at least 90 mol% and a catalyst life of at least 7 kg/kg.

Reference herein to paraffin to olefin ratios are made as ratios by volume unless otherwise stated. By the term paraffin to olefin ratio is meant the quantity of feedstock paraffin per unit quantity of olefin introduced into the reactor.

Reference herein by the term alkylate is to a mixed condensation product of paraffin with olefin(s), and by the term oligomerisation product is to a condensation product of a plurality of olefin molecules. Alkylate is characterised by a higher motor octane number (MON) than oligomerisation product. Typically alkylate comprising highly branched paraffins with 5 to 12 carbon atoms has a MON of 86 or above, for example in the range 90 to 94 whilst a corresponding oligomerisation product may comprise a mixture of paraffinic or olefinic hydrocarbons typically of MON of less than 85, for example in the range 80 to 82.

Internal circulation of liquid reactor content is suitably attained in known reactor types such as for example liquid phase continuous stirred tank reactors or fluidised bed reactors optionally operated with feed cross-flow. Alternatively or additionally circulation may suitably be attained by means of a fluid pump. An ideal continuous stirred tank reactor has infinite distribution of residence time, i.e a variance in residence time distribution of 1. An ideal plug flow reactor has no distribution of residence time, i.e a variance in residence time distribution of 0. Other reactors which may be used in the process of the invention are characterised by an extent of internal circulation which is conveniently expressed in terms of variance of residence time distribution enabling comparison of extent of internal circulation of different reactors. In the process of the invention, liquid reactor contents consists essentially of upgraded product, that is unreacted paraffin and alkylate. It is possible to operate with internal circulation at a high variance in residence time distribution since the internally circulated alkylate appears to be essentially stable under the applied process conditions to further reaction in the presence of catalyst.

The variance in residence time distribution of a reactor is calculated from the residence time distribution for normalised time. The value of variance may thus be obtained for any residence time distribution curve whereby deviation from ideality does not affect comparison with variance of other reactors.

The residence time distribution is the probability that an element of liquid reactor content will leave the reactor after a certain residence time, i.e a measure of the time required to change the complete volume of a reactor. Residence time distribution may be determined by known techniques. Suitably, by pulse injection of a tracer material into the liquid reactor contents under the desired internal circulation conditions and detection of the tracer concentration with each time unit in the reactor effluent, residence time distribution is plotted as the response to a pulse disturbance against time. The variance in the residence time distribution may then be obtained as an integral of the obtained plot, suitably the variance $\sigma_\theta^2$ is equal to the integral

$$\int_0^\infty (\theta - \bar{\theta})^2 \; E(\theta).d\theta,$$

wherein E is the residence time distribution function of a reactor, $\theta$ is mean residence time expressed in reduced time, $\bar{\theta}$ is reduced time and wherein reduced time is the ratio of measured time to holding time of the total reactor flow, holding time being the inverse of reactor flow per unit reactor volume. Suitable tracer materials may be in the form of an isotopic, coloured or otherwise detectable label introduced into the reactor contents. For example, olefin may be introduced as tracer into a reactor containing paraffin and in place of catalyst an inert but otherwise similar material. Olefin concentration is suitably measured by means of chromatography of the effluent stream.

The process may be carried out in a single reactor or in a plurality of reactors in series as a cascade. A cascade of reactors may be housed in a single unit or in separate units. Increased capacity may be achieved by operation of a cascade of reactors in parallel. The conditions according to the process of the invention may be attained in other known reactor types.

Olefin conversion is defined as the ratio of olefin consumed in the reactor to the olefin introduced. At a high level of olefin conversion the desired product alkylate is obtained. High olefin conversion levels may be achieved by use of appropriate olefin space velocities suitably below 10 kg/kg.h and high activity catalysts together with appropriate reactor temperature, composition and rate of introduction of paraffin and olefin and removal of upgraded products during the reaction. The level of olefin conversion is conveniently measured by gas chromatographic techniques in the effluent of the reactor.

Preferably the process of the invention is operated with a strongly acidic solid acid catalyst having a high acid site density and/or strongly acidic acid sites. The process is advantageously operated at an olefin space velocity at which at least 90mol% olefin conversion is obtained. Olefin space velocity is defined as the weight of olefin introduced into the reactor per unit time per unit weight of catalyst in the reactor. For example a strongly acidic catalyst may be used in the process of the invention at an olefin space velocity 10 kg/kg.h whilst a less acidic catalyst may be used at olefin space velocities advantageously below 0.1 and above 0.01 kg/kg.h. Nevertheless operating conditions may be selected as required whereby olefin space velocity is increased above the optimum for ease of operation at sacrifice of some catalyst life. Catalyst life may be defined as the catalyst age at which deactivation is substantially complete. Catalyst age is defined as weight olefin per unit catalyst weight, this being calculated as the product of olefin space velocity and time on stream. It has been found that the process of the invention operates at high catalyst ages before deactivation of the catalyst takes place. Operation beyond a catalyst life of 7 kg/kg and up to lives of the order of 15 kg/kg has been attained.

As a further advantage of the invention, operation at high olefin conversion levels in an internal circulation reactor according to the invention results in high alkylate yields selective to highly branched alkylates throughout the progressive deactivation of the catalyst. Only at full deactivation has olefin breakthrough been observed. Thus it may be economical to operate the process for the full catalyst life.

Excellent results are achieved with the process of the invention when operating in a single reactor or in a cascade of reactors with split feed to each reactor. Suitably up to ten reactors may be used, preferably up to eight and most preferably five. Feed supply to each operating reactor of a cascade suitably comprises individual feed lines supplied from a central feed line.

A preferred operation of the process of the invention is in the preparation of highly branched paraffins containing 5 to 12 carbon atoms, preferably containing 5,6,7,8,9 or 12 carbon atoms, most preferably trimethyl-butane, -pentanes or -hexanes. The process may be applied in the upgrading of a paraffinic feedstock comprising iso-paraffins having from 4 to 8 carbon atoms as desired, suitably of a feedstock comprising isobutane, 2-methylbutane, 2,3-dimethylbutane, 3-methylhexane or 2,4-dimethylhexane, most preferably a paraffinic feedstock comprising iso-paraffins having 4 or 5 carbon atoms. Suitable feedstocks for the process of the invention include iso-paraffin containing fractions of oil conversion products such as naphtha fractions, and refined iso-paraffin feedstocks such as refined iso-butane. It is convenient to isolate the iso-paraffin content of the effluent upgraded product for re-use as feedstock.

The olefins-containing stream is suitably in the form of a lower olefin containing hydrocarbon which may optionally contain additional non-olefinic hydrocarbons. Suitably the olefins-containing stream is ethene, propene, iso- or 1- or 2-(cis or trans) butene or pentene optionally diluted for example with propane, iso-butane, n-butane or pentanes. The process of the invention may suitably be operated downstream of a fluid catalytic cracking unit, an MTBE etherification unit or an olefin isomerisation unit.

Preferably the reactor is started up in such a manner as to control initial contact of the catalyst with olefin feed. Suitably the reactor is first charged with paraffinic feedstock. Required internal circulation conditions are attained and an olefins-containing stream is subsequently continuously and quantitatively charged together with the feedstock at an olefin space velocity such that conversion is maintained in excess of 90 mol%. The feedstock and olefins stream may advantageously be charged via a plurality of inlet ports for example in cross-flow configuration. The use of nozzles to charge the reactor may advantageously provide improved dispersion of charge at the site of introduction. Suitable nozzles are those known for use in alkylation reactions using hydrogen fluoride as catalyst and nozzles known for dispersion of feed in stirred tank reactors and fluidised bed reactors. A cascade of reactors may be operated by use of the effluent from a first reactor as the feedstream for the subsequent reactor in the cascade.

The alkylation reaction is carried out at a temperature less than 150 °C, more preferably between 60 and 120 °C. Reaction pressure may be between 1 and 40 atmospheres. Suitably the pressure is above the vapour pressure of the reactor contents so as to maintain the reaction in the liquid phase.

Preferred paraffin to olefin ratio is in excess of 5 v/v, more preferably in the range of 10 to 30 v/v, most preferably in the range of 15 to 30 v/v. A low paraffin to olefin ratio suitably within the range 2 to 10 v/v favours the formation of higher alkylates, such as isoparaffins containing 12 carbon atoms.

The process of the invention preferably has a catalyst life in excess of 7.5 kg/kg, more preferably in excess of 8 kg/kg, most preferably in excess of 9 kg/kg. Catalyst lives in excess of 12.5 kg/kg may be attained with the process of the invention.

Preferred olefin conversion substantially throughout the reactor is at least 95 mol%, more preferably at least 98 mol%, more preferably at least 99 mol%, such as for example 99.5, 99.8 or 99.9 mol%. Most preferably olefin conversion is substantially complete, i.e substantially 100%.

Preferably a reactor may be employed having a variation in residence time distribution of liquid reactor contents greater than 0.5, more preferably greater than 0.75 for example in the range 0.75 to 1.0 and more preferably in the range 0.85 to 1.0, most preferably substantially 1.0.

Operating ranges of olefin space velocity include from 0.01 to 10 kg/kg.h, preferably from 0.01 to 0.50 or from 0.5 to 10 kg/kg.h, most preferably from 0.05 to 0.10 or from 1.0 to 10 kg/kg.h depending on the relative acid strength of the catalyst.

The process may advantageously be operated by application of Advanced Process Control and optionally On-line Optimisation techniques using respectively multi-variable dynamic and static models for the constraint control of selected process parameters. In a preferred operation of the process of the invention the alkylate obtained is monitored during operation and quality data together with the relevant operational process data are fed to an advanced process control system. Real time solutions of the advanced process controller are automatically fed back to a process implementation system and may be implemented in order to ensure process parameters remain within operating constraints. Beneficial results may be obtained by maintaining process parameters such as residence time distribution, temperature and olefin space velocity within predetermined constraints.

The process of the invention may be operated by use of any solid acid catalyst. Particularly suitable catalysts include strongly acidic wide pore molecular sieves such as zeolites of pore diameter greater than 0.60 nm, sulphate mounted metal oxides, amorphous silica alumina or silica magnesia, metal halides on alumina, macroreticular acid cation exchange resins such as acidified perfluorinated polymers, heteropoly compounds and activated clay minerals.

Examples of zeolite catalysts include zeolite beta, zeolite omega, mordenite and faujasites, particularly zeolites X and Y, of which zeolite beta is most preferred. Zeolites may be ion exchanged for example with ammonium ions and thereafter calcined; trivalent rare earth elements such as lanthanum or cerium or mixtures thereof; or divalent alkaline earth ions such as magnesium or calcium to promote acidity. Examples of sulphate mounted metal oxides include those of zirconium oxide, titanium oxide and iron oxide. Preferred is sulphate mounted zirconium oxide. Amorphous silica aluminas may be acidified such as fluorinated or chlorinated amorphous silica alumina. Examples of metal halides on alumina include hydrochloric acid treated aluminium chloride and aluminium chloride on alumina. Examples of cation exchange resins include Nafion (Trade Mark) an acidified perfluorinated polymer of sulphonic acid. Examples of heteropoly compounds include $H_3PW_{12}O_{40}$, $H_4SiMo_{12}O_{40}$ and $H_4SiW_{12}O_{40}$. Examples of clay minerals include smectite and montmorillonite.

Catalysts are suitably present on a support. For example zeolites suitably further comprise a refractory oxide that serves as binder material such as alumina, silica-alumina, magnesia, titania, zirconia and mixtures thereof. Alumina is specially preferred. The weight ratio of refractory oxide and zeolite suitably ranges from 10:90 to 90:10, preferably from 50:50 to 15:85.

Without wishing to be bound by any particular theory it would appear that the acidity of the selected catalyst is dependent on both the density of acid sites in the catalyst structure and on the acid strength of those sites. The acid site density may be determined by titration for example with butylamine, or by NMR or IR techniques. A high acid site density may be beneficial in prolonging the catalyst life prior to deactivation. A highly acidic catalyst may advantageously be used in the process of the invention at high olefin space velocities resulting in increased rate of production of alkylate, whilst a weakly acidic catalyst is preferentially operated at a low space velocity if product alkylate is to be obtained.

Although the acid strength of sites can be determined as such it is convenient to use known techniques for comparing catalyst acid site strengths as given for example in relation to zeolite catalysts in "Introduction to Zeolite Science and Practice", van Bekkum, Flanigen, Jansen, Elsevier 1991 at pages 268 to 278.

It may be desired to increase the potential catalyst life prior to use by increasing the availability of acid sites at which reaction may take place. Suitably a solid acid catalyst may be treated to increase the acid site density by so-called secondary techniques known in the art. A zeolite based catalyst may be treated for example with alkali wash such as sodium hydroxide to redistribute the alumina within the catalyst, decreasing the lattice silica-alumina ratio.

By the process of the invention it is possible to operate at an acceptable selectivity, i.e. obtaining an acceptable alkylate yield for the duration of the time on stream. By this means the catalyst alkylation activity is upheld throughout partial deactivation and until full deactivation of all sites has occurred, at which point olefin breakthrough is observed. Breakthrough is conveniently indicated by gas chromatography. Hence for the duration of time on stream it would appear that oligomerisation product remains associated with the acid sites of the catalyst and is not detected in the product.

Regeneration of the catalyst may be carried out by techniques known in the art for desorbing hydrocarbonaceous deposits from acid sites, such as oxidative regeneration at elevated temperature in oxygen-rich atmosphere. The process of the invention is characterised by increased efficiency in cases in which the regeneration rate is greater than the deactivation rate. Hence techniques which increase the regeneration rate may be of particular advantage in the process.

Regeneration may be carried out continuously, for example one or more of a cascade of reactors may be operated as desired in reaction or in regeneration mode. Fresh or regenerated catalyst may be continuously added to an independent reactor or each of a cascade of reactors simultaneously with continuous catalyst withdrawal from the reactor and the withdrawn catalyst passed to a regeneration reactor.

It may be desirable to convert branched alkylate further to other products. Accordingly the process of the invention may include a feed line to a further process unit.

The process is now illustrated by an example.

EXAMPLE 1

Zeolite-beta having pellet size of 0.1 mm and a silica to alumina ratio of 12.7 was introduced into a stirred autoclave which was filled up with liquid isobutane feedstock. The reactor contents were heated up to 100 °C. A mixture of 2-butene and isobutane feedstock (paraffin olefin ratio 30 v/v) was fed into the reactor at an olefin space velocity of 0.05 kg/kg.h. The reactor was operated with a residence time distribution of substantially 1.0, i.e at substantially ideal stirred flow conditions. Alkylate was produced for 250 hours. Olefin conversion was greater than 99.0 mol%, ie substantially complete. The results are given in Table 1 below measured throughout the catalyst life. Deactivation occurred at catalyst age of 12.5 kg/kg.

Yield of essentially paraffinic C5+ product was 200% by weight on olefin.

TABLE 1

| Product | Yield obtained at catalyst ages (kg/kg) | | |
|---|---|---|---|
| | 0.9 | 3.9 | 10.7 |
| C5/C5+ | 17.4 | 9.8 | 7.9 |
| C6/C5+ | 9.3 | 5.8 | 4.7 |
| C7/C7+ | 13.2 | 10.2 | 7.7 |
| C8/C5+ | 59.3 | 69.5 | 76.3 |
| C9+/C5+ | 0.8 + | 4.7 + | 3.4 + |
| | 100 | 100 | 100 |
| TMP/C8 | 55.8 | 68.1 | 69.3 |

**TMP is trimethylpentanes.**

Yields are given as % of total yield in percent of C5+ or C8 as indicated.

COMPARATIVE EXAMPLE

The paraffin and olefin of Example 1 were introduced into a single fixed-bed reactor containing the catalyst of Example 1 and reaction initiated as described in the Example 1 except that no stirring was used, i.e. the variance in residence time distribution of liquid reactor contents was substantially zero. The process was continued up to a catalyst age of 0.5.

Since the catalyst deactivated very rapidly no alkylate was detected in the reactor effluent.

**Claims**

1. Process for upgrading a paraffinic feedstock comprising

   (a) supplying the feedstock and an olefins-containing stream at a paraffin to olefin ratio greater than 2 v/v to a reactor containing a solid acid catalyst and
   (b) removing upgraded product, characterised in that the process is operated in an internal circulation reactor at an olefin space velocity in the range from 0.01 to 10 kg/kg.h, a temperature of less than 150 °C and a variance in residence time distribution of liquid reactor contents of greater than 0.25 such that the process has an olefin conversion of at least 90 mol% and a catalyst life of at least 7 kg/kg.

2. Process according to claim 1 operated at an olefin conversion of at least 95 mol%.

3. Process according to any of claims 1 and 2 wherein the variance in residence time distribution of the liquid reactor contents is greater than 0.5.

4. Process according to any of claims 1 to 3 which has a catalyst life in excess of 9 kg/kg.

5. Process according to any of claims 1 to 4 wherein the catalyst is first contacted with the paraffinic feedstock and subsequently olefin is continuously and quantitatively charged together with the feedstock at an olefin space velocity in the range from 0.01 to 10 kg/kg.h such that conversion maintained is at least 90 mol%.

6. Process according to any of claims 1 to 5 characterised by a paraffin to olefin ratio greater than 10 v/v.

7. Process according to any of claims 1 to 6 operated in one or more of a cascade of reactors in series.

8. Process according to claim 7 wherein one or more reactors are operated in catalyst regeneration mode.

9. Process according to any of claims 1 to 8 operated with continuous catalyst regeneration wherein catalyst is added to and withdrawn from the reactor and the withdrawn catalyst is passed to a regeneration step.

10. Process according to any of claims 1 to 9 wherein steady state operation within residence time distribution, temperature and olefin space velocity ranges as defined in the foregoing claims is achieved by use of Advanced Process Control monitoring alkylate quality together with operational process data.

11. Process according to any of claims 1 to 10 wherein the catalyst is selected from strongly acidic wide pore molecular sieves, sulphate-mounted metal oxides, amorphous silica alumina or silica magnesia, metal halides on alumina, macroreticular acid cation exchange resins, heteropoly compounds and activated clay minerals.

12. Process according to claim 11 wherein the catalyst is selected from crystalline silica alumina zeolites of pore diameter greater than 0.60 nm, metal halides on alumina and acidified perfluorinated polymers.

13. Process according to claim 11 wherein the catalyst is zeolite beta.

14. Process according to any of claims 1 to 13 wherein the catalyst has been treated to increase the acid site density.


**Patentansprüche**

1. Verfahren zum Aufbessern eines paraffinischen Einsatzmaterials, umfassend:

a) Zuführen des Einsatzmaterials und eines olefinhältigen Stroms bei einem Paraffin/Olefin-Verhältnis von größer als 2 V/V zu einem einen festen sauren Katalysator enthaltenden Reaktor und
b) Abführen des aufgebesserten Produktes, dadurch gekennzeichnet, daß das Verfahren in einem Reaktor mit Innenkreislauf bei einer Olefinraumgeschwindigkeit im Bereich von 0,01 bis 10 kg/kg.h, einer Temperatur von weniger als 150°C und einer Varianz der Verweilzeitverteilung des flüssigen Reaktorinhalts von größer als 0,25 derart ausgeführt wird, daß das Verfahren eine Olefinumwandlung von wenigstens 90 Mol-% und eine Katalysatorlebensdauer von wenigstens 7 kg/kg aufweist.

2. Verfahren nach Anspruch 1, das bei einer Olefinumwandlung von wenigstens 95 Mol-% ausgeführt wird.

3. Verfahren nach einem der Ansprüch 1 und 2, worin die Varianz der Verweilzeitverteilung des flüssigen Reaktorinhaltes größer als 0,5 ist.

4. Verfahren nach einem der Ansprüche 1 bis 3, das eine Katalysatorlebensdauer von über 9 kg/kg aufweist.

5. Verfahren nach einem der Ansprüche 1 bis 4, worin der Katalysator zuerst mit dem paraffinischen Einsatzmaterial in Berührung gebracht wird und anschließend das Olefin kontinuierlich und quantitativ zusammen mit dem Einsatzmaterial bei einer Olefinraumgeschwindigkeit im Bereich von 0,01 bis 10 kg/kg.h derart zugeführt wird, daß die Umwandlung auf wenigstens 90 Mol-% gehalten wird.

6. Verfahren nach einem der Ansprüche 1 bis 5, gekennzeichnet durch ein Paraffin/Olefin-Verhältnis von größer als 10 V/V.

7. Verfahren nach einem der Ansprüche 1 bis 6, das in einem Reaktor oder in mehreren, in Reihe angeordneten Reaktoren ausgeführt wird.

8. Verfahren nach Anspruch 7, worin ein oder mehrere Reaktoren im Katalysatorregenerationsmodus betrieben werden.

9. Verfahren nach einem der Ansprüche 1 bis 8, das mit einer kontinuierlichen Katalysatorregenerierung betrieben wird, worin Katalysator dem Reaktor zugeführt und aus diesem abgenommen wird und der abgenommene Katalysator einer Regenerationsstufe zugeführt wird.

10. Verfahren nach einem der Ansprüche 1 bis 9, worin ein Fließgleichgewichtsbetrieb innerhalb Verweilzeitverteilungsbereichen, Temperaturbereichen und Olefinraumgeschwindigkeitsbereichen, wie in den vorstehenden Ansprüchen definiert, durch Anwendung von fortgeschrittener Verfahrensregelung unter Verfolgung der Alkylatqualität zusammen mit den Verfahrensbetriebsdaten erzielt wird.

11. Verfahren nach einem der Ansprüche 1 bis 10, worin der Katalysator unter stark sauren großporigen Molekularsieben, auf Sulfat aufgebrachten Metalloxiden, amorphem Siliciumdioxid-Aluminiumoxid oder Siliciumdioxid-Magnesiumoxid, Metallhalogeniden auf Aluminiumoxid, makroretikularen sauren Kationenaustauscherharzen, Heteropolyverbindungen und aktivierten Tonmineralien ausgewählt ist.

12. Verfahren nach Anspruch 11, worin der Katalysator unter kristallinen Siliciumdioxid-Aluminiumoxid-Zeolithen mit einem Porendurchmesser von größer als 0,60 nm, Metallhalogeniden auf Aluminiumoxid und angesäuerten perfluorierten Polymeren ausgewählt ist.

13. Verfahren nach Anspruch 11, worin der Katalysator β-Zeolith ist.

14. Verfahren nach einem der Ansprüche 1 bis 13, worin der Katalysator zur Steigerung der Säurenstellendichte behandelt worden ist.

## Revendications

1. Procédé pour valoriser ou améliorer une charge de départ paraffinique, conformément auquel

   (a) on introduit la charge de départ et un courant contenant des oléfines à un rapport paraffine à oléfine supérieur à 2 v/v, dans un réacteur contenant un catalyseur acide solide et

   (b) on prélève du produit valorisé ou amélioré, caractérisé en ce que l'on fait fonctionner le procédé dans un réacteur à circulation interne, à une vitesse spatiale d'oléfine qui varie de 0,01 à 10 kg/kg.h, à une température inférieure à 150°C et une variance de la distribution des durées de séjour du contenu du réacteur liquide supérieure à 0,25, en une manière telle que le procédé fournisse une conversion d'oléfine d'au moins 90% molaires et une vie de catalyseur d'au moins 7 kg/ kg.

2. Procédé suivant la revendication 1, entrepris à une conversion d'oléfine d'au moins 95% molaires.

3. Procédé suivant l'une quelconque des revendications 1 et 2, caractérisé en ce que la variance de la distribution des durées de séjour du contenu du réacteur liquide est supérieure à 0,5.

4. Procédé suivant l'une quelconque des revendications 1 à 3, caractérisé en ce que la durée de vie ou vie du catalyseur est supérieure à 9 kg/kg.

5. Procédé suivant l'une quelconque des revendications 1 à 4, caractérisé en ce que l'on met d'abord le catalyseur en contact avec la charge de départ paraffinique et en ce que l'on introduit ensuite de l'oléfine, de manière continue et quantitative ensemble avec la charge de départ, à une vitesse spatiale d'oléfine qui fluctue de 0,01 à 10 kg/kg.h, en sorte que la conversion soit maintenue à au moins 90% molaires.

6. Procédé suivant l'une quelconque des revendications 1 à 5, caractérisé par un rapport paraffine à oléfine supérieur à 10 v/v.

7. Procédé suivant l'une quelconque des revendications 1 à 6, mis en oeuvre dans un ou plusieurs réacteurs d'une cascade de réacteurs raccordés en série.

8. Procédé suivant la revendication 7, caractérisé en ce qu'un ou plusieurs réacteurs fonctionnent dans le mode régénération du catalyseur.

9. Procédé suivant l'une quelconque des revendications 1 à 8, mis en oeuvre avec une régénération de catalyseur continue, où on introduit du catalyseur dans le et on le prélève du réacteur et on fait passer le catalyseur prélevé dans une étape de régénération.

10. Procédé suivant l'une quelconque des revendications 1 à 9, caractérisé en ce que l'on parvient à un fonctionnement à état constant, dans les plages des distributions des durées de séjour, des températures et des vitesses spatiales d'oléfines, telles que définies dans les revendications précédentes, par l'emploi d'un système de réglage de procédé avancé surveillant la qualité de l'alkylat en même temps que les données opératoires du processus.

11. Procédé suivant l'une quelconque des revendications 1 à 10, caractérisé en ce que l'on choisit le catalyseur parmi des tamis moléculaires à larges pores, fortement acides, des oxydes de métaux montés sur sulfate, de la silice-magnésie ou de la silice-alumine amorphe, des halogénures de métaux sur alumine, des résines échangeuses de cations acides macroréticulaires, des hétéropolycomposés et des argiles minérales activées.

12. Procédé suivant la revendication 11, caractérisé en ce que l'on choisit le catalyseur parmi des zéolites à silice-alumine cristallines, d'un diamètre des pores supérieur à 0,60 nm, des halogénures de métaux sur alumine et des polymères perfluorés acidifiés.

13. Procédé suivant la revendication 11, caractérisé en ce que le catalyseur est la zéolite bêta.

14. Procédé suivant l'une quelconque des revendications 1 à 13, caractérisé en ce que le catalyseur a été traité, en vue d'augmenter la densité des sites acides.